# EUROPEAN PATENT APPLICATION

(11) **EP 4 564 009 A1**
(43) Date of publication of application: **04.06.2025**
(21) Application number: 23845975.4
(22) Date of filing: 23.05.2023
(51) Int. Cl.: G01N 35/00

(54) **ANALYSIS SYSTEM AND DATA PROCESSING DEVICE**

(30) Priority: 27.07.2022 JP 2022119473
(71) Applicant: SHIMADZU CORPORATION, Kyoto-shi, Kyoto 604-8511 (JP)
(72) Inventor: SUDO, Tomokazu, Kyoto-shi, Kyoto 604-8511 (JP); SUGIYAMA, Kiyohiro, Kyoto-shi, Kyoto 604-8511 (JP)
(74) Representative: Müller-Boré & Partner Patentanwälte PartG mbB
(86) International application number: PCT/JP2023/019064
(87) International publication number: WO 2024/024237

(57) **Abstract**

The present invention is an analysis system 1 including: an analyzer 10 configured to mix a reagent in a reagent container 114 held in a reagent holder 111 with a specimen sample in a specimen container held in a specimen holder 121 and analyze a mixture; and a data processing device 30 including a temperature information collection unit 310 configured to collect temperature information of the reagent holder via a data communication network 20, wherein the data processing device includes: a display unit 33 configured to display a screen showing the temperature information collected by the temperature information collection unit; a temperature determination unit 313 configured to determine, based on the temperature information, whether a temperature of the reagent holder is out of a predetermined temperature range; and a display control unit 314 configured to cause the screen showing the temperature information to pop up in response to the temperature determination unit determining that the temperature of the reagent holder is out of the predetermined temperature range.

## Description

### TECHNICAL FIELD

The present invention relates to an analysis system including an analyzer and a data processing device connected to the analyzer via a data communication network, and further relates to a data processing device.

### BACKGROUND ART

A biochemical analyzer is one of the devices for analyzing components contained in a specimen of blood, urine, etc. In a biochemical analyzer, a reagent corresponding to a component to be analyzed is mixed with a specimen in a reaction container, and the intensity of transmitted light or scattered light generated by irradiating the mixture with light from a light source is measured using a photodetector. The biochemical analyzer is connected to a personal computer (hereinafter referred to as "PC") as a data processing device, and the intensity of transmitted light or scattered light detected by the photodetector is input to the PC and then converted into the concentration of the component.

In many cases, a component or components contained in a plurality of specimens are analyzed. In this regard, many biochemical analyzers include a specimen rack holder which holds a specimen rack on which a plurality of specimen containers are mounted, a reagent tray holder which holds a reagent tray on which reagent containers are mounted, and a dispensing mechanism which collects a specimen and a reagent from a specimen container and a reagent container, respectively, and injects the specimen and the reagent into a reaction container. Then, the biochemical analyzer automatically and continuously performs a series of operations of sequentially collecting a specimen and a reagent from the specimen containers and the reagent containers, injecting the specimen and the reagent into a reaction container, and measuring light intensity (Patent Literature 1).

Such a biochemical analyzer usually includes a refrigerating mechanism for refrigerating the inside of the reagent tray holder in order to prevent deterioration of the reagents in the reagent containers. When the temperature in the reagent tray holder deviates from an appropriate temperature range due to a defect of the refrigerating mechanism, the reagent in the reagent container is frozen or denatured. In addition, in a case where the reagent tray holder includes a turntable, when the dew condensation water freezes in the vicinity of the turntable due to a temperature drop in the reagent tray holder below an appropriate temperature range, the turntable may not be able to rotate. Therefore, the biochemical analyzer includes a temperature sensor for detecting the temperature in the reagent tray holder so as to display the temperature on a screen of a display unit of the PC connected to the biochemical analyzer.

### CITATION LIST

### PATENT LITERATURE

Patent Literature 1: JP 2018-185345 A

### SUMMARY OF INVENTION

### TECHNICAL PROBLEM

When the biochemical analyzer is performing an analysis operation, the screen of the display unit of the PC predominantly displays information related to analysis (analysis related information) such as a progress status of the analysis operation and an analysis result, and an area for displaying information not directly related to the analysis operation (non-analysis information) such as the temperature in a reagent tray holder is normally smaller than the area displaying the analysis related information, or the display area of the analysis related information overlaps the displaying area of the non-analysis information, so that a part or all of the non-analysis information area is hidden. In addition, in many cases, the display area of the non-analysis information displays only the temperature in the reagent tray holder, and if a failure occurs in the refrigerating mechanism, the failure may be overlooked.

Here, a biochemical analyzer is described as an example, but analyzers having a refrigerating mechanism such as a biochemical analyzer or a blood coagulation analyzer have a similar problem.

An object of the present invention is to provide an analyzer including a reagent holder which holds reagents, the analyzer being configured to prevent overlooking of the temperature of the reagent holder deviating from an appropriate temperature range.

### SOLUTION TO PROBLEM

A mode of the present invention made to solve the above problems is
an analysis system including: an analyzer configured to mix a reagent in a reagent container held in a reagent holder with a specimen sample in a specimen container held in a specimen holder and analyze a mixture; and a data processing device including a temperature information collection unit configured to collect temperature information of the reagent holder via a data communication network, wherein
the data processing device includes:
   a display unit configured to display a screen showing the temperature information collected by the temperature information collection unit;
   a temperature determination unit configured to determine, based on the temperature information, whether a temperature of the reagent holder is out of a predetermined temperature range; and
   a display control unit configured to cause the screen showing the temperature information to pop up in response to the temperature determination unit determining that the temperature of the reagent holder is out of the predetermined temperature range.

Another mode of the present invention made to solve the above problems is
a data processing device connected via a data communication network to an analyzer configured to mix a reagent in a reagent container held in a reagent holder with a specimen sample in a specimen container held in a specimen holder and analyze a mixture, the data processing device including:
a temperature information collection unit configured to collect temperature information of the reagent holder via the data communication network;
a display unit configured to display a screen showing the temperature information collected by the temperature information collection unit;
a temperature determination unit configured to determine, based on the temperature information, whether the temperature of the reagent holder is out of a predetermined temperature range; and
a display control unit configured to cause the screen showing the temperature information to pop up in response to the temperature determination unit determining that the temperature of the reagent holder is out of the predetermined temperature range.

### ADVANTAGEOUS EFFECTS OF INVENTION

According to the present invention, when a failure occurs in a mechanism for refrigerating the reagent holder included in the analyzer, and the temperature of the reagent holder indicates an abnormal value, the temperature information of the reagent holder is popped up on the screen of the display unit. This configuration can prevent the operator of the data processing device from overlooking the abnormal value of the temperature of the reagent holder.

### BRIEF DESCRIPTION OF DRAWINGS

[Fig. 1] A diagram illustrating a schematic configuration of an embodiment of an analysis system.
[Fig. 2] A diagram illustrating a schematic configuration of a reagent refrigerator.
[Fig. 3] A diagram illustrating an example of a temperature information display window displayed on a screen of a display unit of a data processing device.
[Fig. 4] A diagram illustrating an example of an analysis related information display window displayed on the screen of the display unit of the data processing device.
[Fig. 5] A diagram illustrating a state in which the temperature information display window is popped up with the analysis related information display window being displayed on the screen of the display unit of the data processing device.

### DESCRIPTION OF EMBODIMENTS

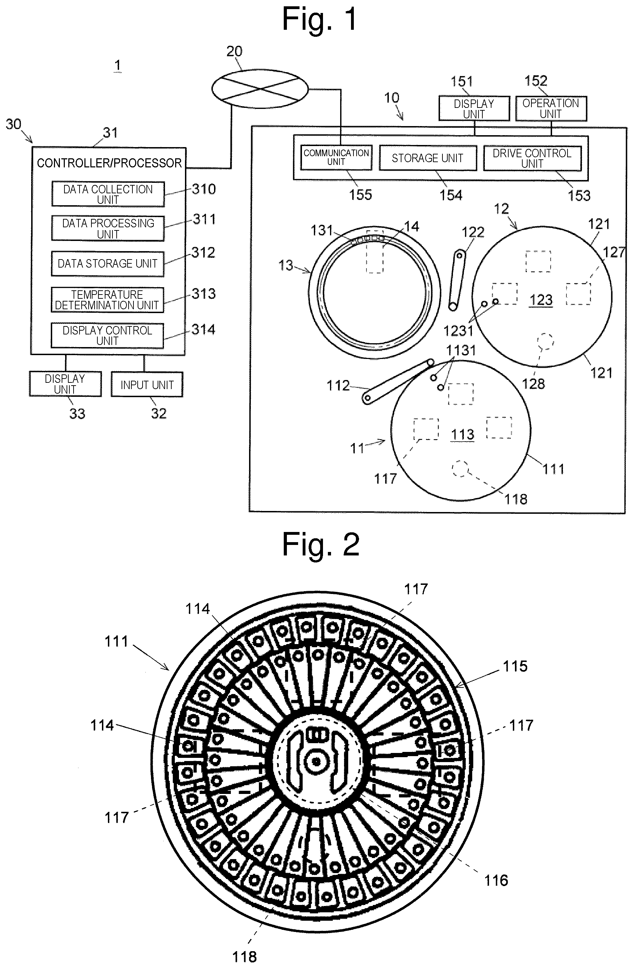

Hereinafter, an embodiment of the present invention will be described with reference to the drawings. Fig. 1 is a schematic configuration diagram of an embodiment of an analysis system. This analysis system 1 according to the present embodiment includes a biochemical analyzer 10 and a data processing device 30 connected to the biochemical analyzer 10 via a wireless or wired data communication network 20. Although only one biochemical analyzer 10 as an analyzer is illustrated in Fig. 1, the number of analyzers constituting the analysis system 1 may be two or more. In a case where the number of analyzers is two or more, all the analyzers may be the same type of analyzer or different types of analyzers.

The biochemical analyzer 10 is a device which automatically measures an amount of a certain component contained in a biological sample of blood, urine, etc (which sample corresponds to a specimen sample in the present invention and is also referred to as specimen). The biochemical analyzer 10 includes a reagent dispensing device 11, a specimen dispensing device 12, a reaction disk 13, and a photometer 14 as main components. The reagent dispensing device 11 includes a reagent refrigerator 111 and a reagent dispensing mechanism 112 which sucks a reagent in the reagent refrigerator 111 and discharges the reagent into a reaction container 131 on the reaction disk 13. The specimen dispensing device 12 includes a specimen refrigerator 121 and a specimen dispensing mechanism 122 which sucks a specimen in the specimen refrigerator 121 and discharges the specimen into the reaction container 131 on the reaction disk 13.

Upper surfaces of the reagent refrigerator 111 and the specimen refrigerator 121 are closed by lids 113 and 123, respectively. The lids 113 and 123 have, respectively, a plurality of suction holes 1131 and 1231 through which an internal liquid to be dispensed is sucked by the reagent dispensing mechanism 112 and the specimen dispensing mechanism 122.

Fig. 2 illustrates a schematic configuration of the reagent refrigerator 111. Since the reagent refrigerator 111 and the specimen refrigerator 121 have substantially the same configuration, only the configuration of the reagent refrigerator 111 will be described here. As illustrated in Fig. 2, a turntable 116 is provided at the bottom of the reagent refrigerator 111, and a reagent tray 115 on which a plurality of reagent containers 114 are mounted is placed on the turntable 116. In the example of the reagent tray 115 illustrated in Fig. 2, the reagent containers 114 are disposed in two rows along the two large and small circumferences about the rotation axis of the turntable 116, and accordingly, the suction holes 1131 (see Fig. 1) of the lid 113 are each formed on the corresponding two large and small circumferences. In the example of the reagent tray 115 illustrated in Fig. 2, different types of reagent containers 114 are mounted along the inner circumference and the outer circumference. In the present embodiment, the reagent refrigerator 111 corresponds to a reagent holder.

In addition, a cooling unit 117 including, for example, a Peltier device and a temperature sensor 118 are disposed in a space below the turntable 116 at the bottom of the reagent refrigerator 111. A first surface of the Peltier device included in the cooling unit 117 is in contact with the inner wall of the reagent refrigerator 111, and a second surface is located outside the reagent refrigerator 111. When the Peltier device is energized, the first surface of the Peltier device absorbs heat, and the absorbed heat moves to the second surface. As a result, the inner wall of the reagent refrigerator 111 in contact with the first surface of the Peltier device is cooled. The temperature sensor 118 detects the temperature in the reagent refrigerator 111. Similarly to the reagent refrigerator 111, the specimen refrigerator 121 also includes a cooling unit 127 including a Peltier device and a temperature sensor 128. Although not illustrated, a temperature sensor for detecting the temperature in the reaction disk 13 is also provided in the vicinity of the reaction disk 13.

The biochemical analyzer 10 includes a display unit 151, an operation unit 152, a drive control unit 153, a storage unit 154, and a communication unit 155. The display unit 151 is, for example, a liquid crystal display. The operation unit 152 includes a keyboard, a touch panel, and the like. The drive control unit 153 controls driving of each unit of the biochemical analyzer 10.

The storage unit 154 stores programs of analysis operations executed by the drive control unit 153 and various parameters. In addition, the storage unit 154 stores a measurement signal of the photometer 14 and detection temperature signals of the temperature sensors 118 and 128. The communication unit 155 transmits the measurement signal of the photometer 14 and the detection signals of the temperature sensors 118 and 128 stored in the storage unit 154 to the data processing device 30 through the data communication network 20.

The data processing device 30 is typically a PC, and includes a controller/processor 31 which processes and stores data (signals) acquired from the biochemical analyzer 10, and an input unit 32 and a display unit 33 attached to the controller/processor 31. The controller/processor 31 includes, as functional blocks, a data collection unit 310 which collects data from the biochemical analyzer 10 connected to the data communication network 20, a data processing unit 311 which processes the data collected by the data collection unit 310, a data storage unit 312 which stores the data processed by the data processing unit 311, a temperature determination unit 313 which determines whether the temperatures in the reagent refrigerator 111 and the specimen refrigerator 121 obtained by processing the detection signals of the temperature sensors 118 and 128 are out of a predetermined temperature range, and a display control unit 314 which controls the display unit 33. Functions of at least a part of the controller/processor 31 is achieved by causing a personal computer (or higher-performance work station) as a hardware resource including a CPU, a memory, and a large-scale storage device such as a hard disk device to execute dedicated control and processing software installed on the computer. In the present embodiment, the data collection unit 310 corresponds to a temperature information collection unit.

Next, operations of the analysis system of the present embodiment will be described.

When the biochemical analyzer 10 performs a specimen analysis operation, first, the Peltier device of the cooling unit 117 of the reagent refrigerator 111 and the Peltier device of the cooling unit 127 of the specimen refrigerator 121 are energized. This operation cools the inside of each refrigerator. At this time, the detection signals of the temperature sensors 118 and 128 included in the reagent refrigerator 111 and the specimen refrigerator 121 are stored in the storage unit 154, and then transmitted to the data processing device 30 via the communication unit 155 and the data communication network 20 at an appropriate timing. The detection signals of the temperature sensors 118 and 128 input to the data processing device 30 are converted into temperatures in the data processing unit 311. Similarly, the detection signal of the temperature sensor disposed in the vicinity of the reaction disk 13 is also converted into a temperature in the data processing unit 311. The converted temperature values are stored in the data storage unit 312 as the respective temperatures inside the reagent refrigerator 111, the specimen refrigerator 121, and the reaction disk 13, and are displayed in a temperature display window 331 displayed on the display unit 151 by the display control unit 314.

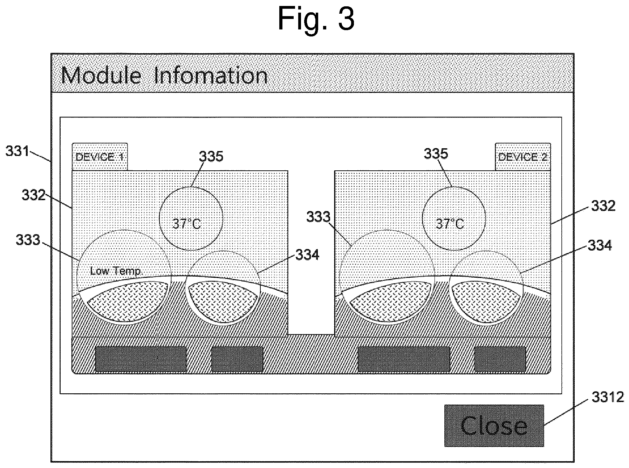

Fig. 3 illustrates an example of the temperature display window 331. In the temperature display window 331, a rectangular graphic 332 indicating the biochemical analyzer 10 connected to the data processing device 30, and circular graphics 333 to 335 indicating, respectively, the reagent dispensing device 11, the specimen dispensing device 12, and the reaction disk 13 included in the biochemical analyzer 10 are illustrated together with information indicating temperatures. Fig. 3 illustrates an example of the temperature display window 331 displayed on the display unit 33 in a case where two biochemical analyzers 10 are connected to the data processing device 30.

When the data processing unit 311 converts the detection signals of the temperature sensors 118 and 128 into temperatures, the temperature determination unit 313 determines whether the temperatures are within a predetermined temperature range. Then, when the temperature determination unit 313 determines that the temperatures detected by the temperature sensors 118 and 128 are normal values within the predetermined temperature range, the display control unit 314 displays nothing in the graphic 333 representing the reagent dispensing device 11 or in the graphic 334 representing the specimen dispensing device 12. The control of the display control unit 314 will be described later in a case where it determines that the detected temperature is an abnormal value out of the predetermined temperature range.

When the analysis operation of the biochemical analyzer 10 is started by the operation of the user, the turntables 116 of the reagent refrigerator 111 and the specimen refrigerator 121, the reaction disk 13, the reagent dispensing mechanism 112, and the specimen dispensing mechanism 122 are driven and controlled by the signal supplied from the drive control unit 153, and these units are moved accordingly and the analysis operations on the specimens contained in the specimen containers are sequentially executed.

Specifically, the specimen containers 124 are sequentially positioned immediately below a suction hole 1231 by driving of the turntable, and the probe at the tip of the specimen dispensing mechanism 122 enters the specimen refrigerator 121 through the suction hole 1231, sucks the specimen in a positioned specimen container 124, and then moves immediately above the reaction container 131 positioned at a specimen discharge position by driving of the reaction disk 13 to discharge the specimen. In the reagent dispensing device 11, similarly to the above, the reagent dispensing mechanism 112 sucks the reagent in a reagent container 114 positioned immediately below a suction hole 1131 by driving of the turntable 116, and discharges the reagent into the reaction container 131 positioned at a reagent discharge position on the reaction disk 13.

When the specimen and the reagent are sequentially dispensed into each reaction container 131 on the reaction disk 13 in this manner, the components in the specimen and the reagent react in the reaction container 131 by driving of the reaction disk 13, and then, the reaction container 131 is sent to the photometer 14. The photometer 14 includes, for example, a spectrophotometer, and measures optical characteristics of the reaction liquid in the reaction container 131. The result (measurement signal) measured by the photometer 14 is stored in the storage unit 154 and then transmitted to the data processing device 30 via the communication unit 155 and the data communication network 20 in response to a data transmission request from the data processing device 30.

The measurement signal of the photometer 14 input to the data processing device 30 is converted into a component concentration and then analyzed by the data processing unit 311. The analyzed result is stored in the data storage unit 312 as information related to analysis, and is displayed in an analysis information display window 336 displayed on the display unit 151 by the display control unit 314.

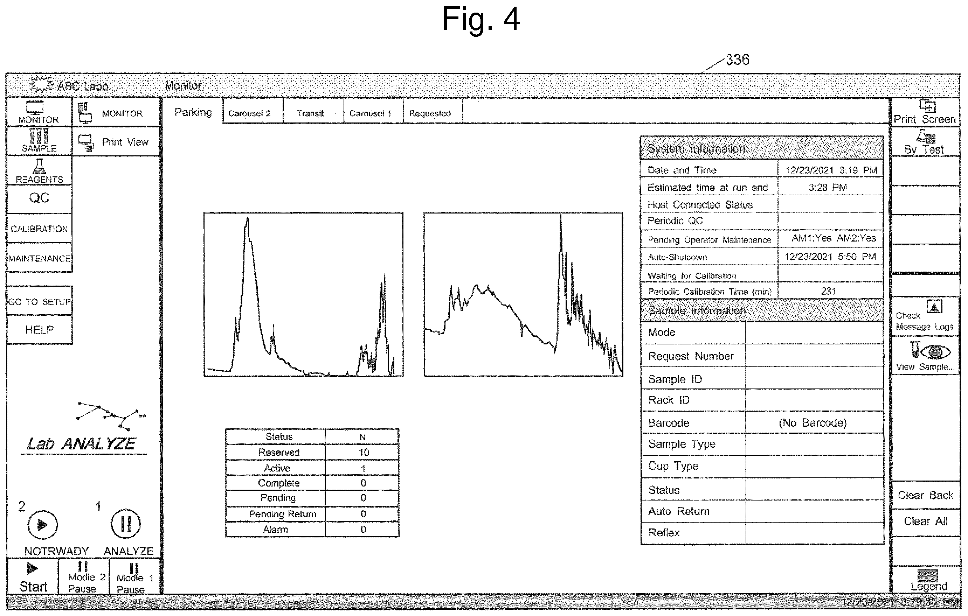

Fig. 4 illustrates an example of the analysis information display window 336. In the analysis information display window 336, the results analyzed by the data processing unit 311 are displayed in various formats such as a table and a graph. The analysis information display window 336 includes a display area in which a progress status of the analysis operation is displayed, an operation area in which graphical user interfaces (GUIs) representing instruction input buttons related to the analysis operation are shown, and the like, in addition to the area in which the analysis result is displayed. When the analysis information display window 336 is displayed on the display unit 33 of the data processing device 30 and the temperature determination unit 313 determines that the temperatures detected by the temperature sensors 118 and 128 are normal values (normal state), the analysis information display window 336 is predominantly displayed on the screen displayed on the display unit 33, and the temperature display window 331 is hidden behind the window 336 and cannot be seen.

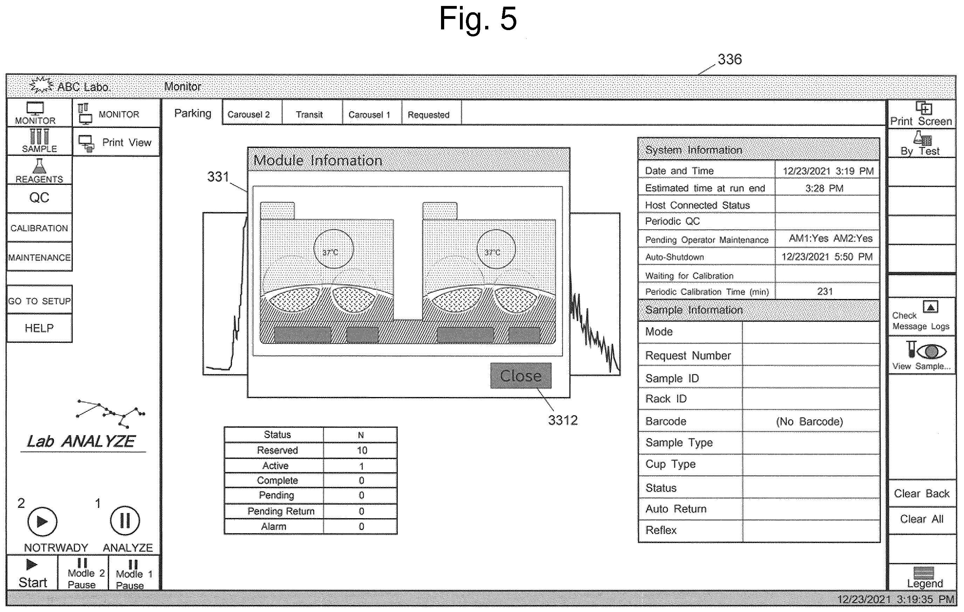

In this state, when the temperature determination unit 313 determines that any of the temperatures detected by the temperature sensor 118 or 128 is an abnormal value (abnormal state), the display control unit 314 causes the temperature display window 331 to pop up. Here, "causing the temperature display window 331 to pop up" means that the temperature display window 331 is displayed such that the temperature display window 331 is superimposed on the window displayed on the display unit 33. Since the analysis information display window 336 is displayed on the display unit 33 in this example, the temperature display window 331 is superimposed on the window 336 and displayed as illustrated in Fig. 5.

When the display control unit 314 causes the temperature display window 331 to pop up, the display control unit 314 causes character information (in the example of Fig. 3, "Low Temp.") indicating an abnormal value to be displayed inside the graphic corresponding to one of the temperatures detected by the temperature sensors 118 and 128 which is determined to be an abnormal value.

Therefore, when the temperature display window 331 is popped up, the user can notice that the temperature of either one or both of the reagent dispensing device 11 and the specimen dispensing device 12 is in an abnormal state. Furthermore, since the abnormal state is displayed as character information in the temperature display window 331, the user can recognize whether the temperature value of either one or both of the reagent dispensing device 11 and the specimen dispensing device 12 is lower or higher than the predetermined temperature range. Note that a GUI 3312 representing a "Close" button is displayed in the popped-up temperature display window 331, and selecting and operating the GUI 3312 can close the temperature display window 331 to hide the temperature display window 331 behind the analysis information display window 336.

### [Modifications]

In the above embodiment, when one or both of the temperatures detected by the temperature sensors 118 and 128 are determined to be an abnormal value, character information is displayed in the temperature display window 331 to indicate the abnormal value. However, the colors of the graphics representing the reagent dispensing device 11 and the specimen dispensing device 12 may be changed, or such a change of the colors and the character information may be combined. By changing the color, the user can easily notice the abnormal state.

In the above embodiment, the temperature determination unit 313 is configured to determine the abnormal value of both of the temperatures detected by the temperature sensors 118 and 128. Alternatively, the temperature determination unit 313 may only determine whether the temperature detected by the temperature sensor 118, that is, the temperature in the reagent refrigerator 111 is an abnormal value, and the temperature display window 331 may be popped up when the temperature determination unit 313 determines that the temperature is an abnormal value.

Some analyzers are configured to hold the reagent containers at a temperature lower than room temperature and hold the specimen containers at room temperature. Such an analyzer does not need to include a cooling unit including a Peltier device or the like or a temperature sensor in the specimen holder. In this case, the temperature determination unit determines whether the temperature detected by the temperature sensor provided in the reagent holder is out of the predetermined temperature range.

### [Modes]

A person skilled in the art can understand that the previously described illustrative embodiment is a specific example of the following modes of the present invention.

(Clause 1) A mode of the present invention is
an analysis system including: an analyzer configured to mix a reagent in a reagent container held in a reagent holder with a specimen sample in a specimen container held in a specimen holder and analyze a mixture; and a data processing device including a temperature information collection unit configured to collect temperature information of the reagent holder via a data communication network,
wherein the data processing device includes:
   a display unit configured to display a screen showing the temperature information collected by the temperature information collection unit;
   a temperature determination unit configured to determine, based on the temperature information, whether a temperature of the reagent holder is out of a predetermined temperature range; and
   a display control unit configured to cause the screen showing the temperature information to pop up in response to the temperature determination unit determining that the temperature of the reagent holder is out of the predetermined temperature range.

(Clause 3) Another mode of the present invention is
a data processing device connected via a data communication network to an analyzer configured to mix a reagent in a reagent container held in a reagent holder with a specimen sample in a specimen container held in a specimen holder and analyze a mixture, the data processing device including:
a temperature information collection unit configured to collect temperature information of the reagent holder via the data communication network;
a display unit configured to display a screen showing the temperature information collected by the temperature information collection unit;
a temperature determination unit configured to determine, based on the temperature information, whether the temperature of the reagent holder is out of a predetermined temperature range; and
a display control unit configured to cause the screen showing the temperature information to pop up in response to the temperature determination unit determining that the temperature of the reagent holder is out of the predetermined temperature range.

According to the analysis system according to clause 1 or the data processing device according to clause 3, when the temperature determination unit determines that the temperature of the reagent holder is out of the predetermined temperature range, that is, the temperature is abnormal, the screen showing the temperature information is popped up on the display unit included in the data processing device. This configuration allows the user to easily notice that the temperature of the reagent holder is abnormal even when another screen is displayed on the display unit. Thus, it is possible to prevent deterioration of the reagent due to continuation of a state in which the temperature of the reagent holder indicates an abnormal value.

(Clause 2) An analysis system according to clause 2 is the analysis system according to clause 1, wherein
the display control unit is configured to cause a screen including character information showing that the temperature of the reagent holder is out of a predetermined temperature range to pop up on the display unit as the screen showing the temperature information.

(Clause 4) A data processing device according to clause 4 is the data processing device according to clause 3, wherein
the display control unit is configured to cause a screen including character information showing that the temperature of the reagent holder is out of a predetermined temperature range to pop up on the display unit as the screen showing the temperature information.

According to the analysis system according to clause 2 or the data processing device according to clause 4, the user who looks at the screen showing the temperature information popped up on the display unit can recognize that the temperature of the reagent holder is out of the predetermined temperature range with the character information, and thus can clearly recognize that the temperature is an abnormal value.

### REFERENCE SIGNS LIST

1... Analysis System
10... Biochemical Analyzer
11... Reagent Dispensing Device
111... Reagent Refrigerator
112... Reagent Dispensing Mechanism
114... Reagent Container
115... Reagent Tray
116... Turntable
117... Cooling Unit
118... Temperature Sensor
12... Specimen Dispensing Device
121... Specimen Refrigerator
122... Specimen Dispensing Mechanism
127... Cooling Unit
13... Reaction Disk
131... Reaction Container
14... Photometer
20... Data Communication Network
30... Data Processing Device
31... Controller/Processor
310... Data Collection Unit
311... Data Processing Unit
312... Data Storage Unit
313... Temperature Determination Unit
314... Display Control Unit
32... Input Unit
33... Display Unit
331... Temperature Display Window
3312... GUI
336... Analysis Information Display Window

## Claims

1. An analysis system comprising: an analyzer configured to mix a reagent in a reagent container held in a reagent holder with a specimen sample in a specimen container held in a specimen holder and analyze a mixture; and a data processing device including a temperature information collection unit configured to collect temperature information of the reagent holder via a data communication network,
wherein the data processing device includes:
a display unit configured to display a screen showing the temperature information collected by the temperature information collection unit;
a temperature determination unit configured to determine, based on the temperature information, whether a temperature of the reagent holder is out of a predetermined temperature range; and
a display control unit configured to cause the screen showing the temperature information to pop up in response to the temperature determination unit determining that the temperature of the reagent holder is out of the predetermined temperature range.

2. The analysis system according to claim 1, wherein the display control unit is configured to cause a screen including character information showing that the temperature of the reagent holder is out of a predetermined temperature range to pop up on the display unit as the screen showing the temperature information.

3. A data processing device connected via a data communication network to an analyzer configured to mix a reagent in a reagent container held in a reagent holder with a specimen sample in a specimen container held in a specimen holder and analyze a mixture, the data processing device comprising:
a temperature information collection unit configured to collect temperature information of the reagent holder via the data communication network;
a display unit configured to display a screen showing the temperature information collected by the temperature information collection unit;
a temperature determination unit configured to determine, based on the temperature information, whether the temperature of the reagent holder is out of a predetermined temperature range; and
a display control unit configured to cause the screen showing the temperature information to pop up in response to the temperature determination unit determining that the temperature of the reagent holder is out of the predetermined temperature range.

4. The data processing device according to claim 3, wherein the display control unit is configured to cause a screen including character information showing that the temperature of the reagent holder is out of a predetermined temperature range to pop up on the display unit as the screen showing the temperature information.
